(19) Europäisches Patentamt
European Patent Office
Office européen des brevets

(11) EP 1 481 017 B1

(12) EUROPEAN PATENT SPECIFICATION

(45) Date of publication and mention of the grant of the patent:
**02.08.2006 Bulletin 2006/31**

(21) Application number: **02789018.5**

(22) Date of filing: **17.12.2002**

(51) Int Cl.:
***C08G 63/672*** (2006.01) ***C08G 63/12*** (2006.01)
***C08G 63/85*** (2006.01) ***A61L 31/14*** (2006.01)
***A61K 9/16*** (2006.01)

(86) International application number:
**PCT/NL2002/000840**

(87) International publication number:
**WO 2003/072631 (04.09.2003 Gazette 2003/36)**

# (54) BIODEGRADABLE POLYMERIC MATERIAL FOR BIOMEDICAL APPLICATIONS

BIOABBAUBARES POLYMERMATERIAL FÜR BIOMEDIZINISCHE ANWENDUNGEN

MATERIAU POLYMERIQUE BIODEGRADABLE POUR APPLICATIONS BIOMEDICALES

(84) Designated Contracting States:
**AT BE BG CH CY CZ DE DK EE ES FI FR GB GR IE IT LI LU MC NL PT SE SI SK TR**

(30) Priority: **26.02.2002 EP 02075764**

(43) Date of publication of application:
**01.12.2004 Bulletin 2004/49**

(73) Proprietor: **Chienna B.V.**
**3723 MD Bilthoven (NL)**

(72) Inventors:
- **BEZEMER, Jeroen, Mattijs**
  **NL-3515 BG Utrecht (NL)**
- **ROOSMA, Jorg, Ronald**
  **NL-3511 NR Utrecht (NL)**
- **VAN DIJKHUIZEN-RADERSMA, Riemke**
  **NL-3705 BV Zeist (NL)**
- **DE WIJN, Joost, Robert**
  **NL-6522 BP Nijmegen (NL)**

(74) Representative: **Winckels, Johannes Hubertus F. et al**
**Vereenigde**
**Johan de Wittlaan 7**
**2517 JR Den Haag (NL)**

(56) References cited:
**EP-A- 0 830 859**
**WO-A-01/10928**
**US-A- 4 463 165**
**US-A- 4 725 483**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 481 017 B1

## Description

The invention relates to a medical device comprising a polymeric material and the use of the medical device for the preparation of a medicament for guided tissue, as a scaffold for engineering tissue in vitro, or as a matrix for controlled release of a (bio)active substance.

There exists currently a continuously rising interest for new materials which are useful as carriers of biologically active agents and may be used for delivery of such agents *in vivo.* In particular, a lot of research has been dedicated to the design of such carriers which demonstrate a specific degradability profile *in vivo,* by which a desired release pattern of the incorporated biologically active agent is achieved.

One of the rationales behind this research is the desire to develop delivery systems which prolong the release time of existing drugs (i.e. sustained release). Another rationale is the desire to develop systems that obviate or mitigate the poor pharmacokinetic profiles of some drugs. In particular, peptides and proteins cause pharmacokinetic difficulties. Such substances must often be administered parentarelly is a systemic action is required. Also, many drugs have short half lives, which necessitates frequent injection schedules. Patient compliance and the high cost associated with frequent administering protocols provide strong stimuli to find new ways of administration, and concurrently new dosage forms for such drugs. Yet another rationale behind the search for new drug delivery vehicles is the wish to accomplish that an administered biologically active agent, such as a drug, is released at a specific location in the patient. For instance, when administered orally, it may be necessary to design a delivery vehicle that is capable of withstanding the harsh conditions encountered in the stomach, and pass in intact form into the intestines to only there release an incorporated drug (i.e. delayed release).

Polymeric systems which are presently under investigation as drug delivery matrices include a wide variety of biodegradable compounds. A frequently used system is based on polylactic acid (PLA), or on copolymers of polylactic acid and glycolic acid.. Such copolymers are known as PLGA polymers. In the past, microshperes of PLGA polymers have been prepared encapsulating a biologically active agent. However, a number of serious disadvantages is associated with these systems. Amongst these is the poor possibilities for manipulating the release of a protein encapsulated by PLGA microspheres. Diffusion, which plays a crucial role in that respect, of proteins in PLGA matrices is very limited. The release of the incorporated proteins is accordingly completely dependent on the diffusion of the protein through the pores of the matrix (if present), and on the degradation or dissolution of the PLGA *in vivo.* Degradation of the PLGA matrix causes a relatively sharp decrease in pH in the polymeric matrix, which may be harmful to many proteins.

The European patent application 0 830 859 discloses a drug delivery system based on a copolymer of a polyalkylene glycol, preferably polyethylene glycol, and an aromatic polyester, preferably polybutylene terephthalate. This polymeric material has hydrogel-like properties and advantageously enables diffusion of even large molecules, such as proteins, through the polymeric matrix. Furthermore, the biocompatibility of this polymeric material has been found to be very good.

For some purposes, however, it has been found that the degradation of the polymer is relatively slow. As a consequence, traces or particles of the polymeric matrix may remain intact for some time after release of an incorporated drug is complete. Accordingly, there is a desire for provision of a polymeric material, suitable for use as a delivery vehicle for biologically active agents such as drugs and proteins, wherein the degradability characteristics of the polymer can be altered substantially while substantially retaining the excellent biological characteristics of the copolymer which serves as a starting point.

Another trend in developing biomaterials is triggered by the relatively new field of tissue engineering. Typically, in tissue engineering a matrix, often referred to as scaffold, is provided with cells *in vitro* and the combined system of cells and scaffold is implanted into the body of a patient in need of tissue repair. In the beginning, after implantation, the scaffold is intended to provide the mechanical properties and integrity to keep the implant together. Once the cells start to develop and form *de novo* tissue, the scaffold is intended to degrade so that the new tissue can take over its function. Sometimes, the cells are subjected to a culturing protocol *in vivo* to accelerate the process of formation of new tissue *in vivo.*

The ideal material to serve as scaffold to be provided with cells in tissue engineering closely resembles the properties, in particular the mechanical properties, of the tissue which is in need of repair. Because some types of tissue require stronger and stiffer mechanical properties than others, it is desired to have a biodegradable and biocompatible material of which the mechanical properties may be advantageously adjusted to the need of the situation in tissue engineering. Also, depending on the type of tissue to be repaired, a slower or faster degradation of the scaffold may be desired.

It is therefore a goal of the present invention to provide for a medical device comprising a biomaterial which degrades over a favourably short period of time, while at the same time having excellent characteristics with respect to biocompatibility.

Another goal of the present invention is to provide for a medical device comprising a material which has a composition that allows for the adjustment of the degradation characteristics, while the biocompatibility and other characteristics related to the use of the material, for instance as drug delivery vehicle, are substantially not negatively affected by said adjustment.

It is also a goal of the present invention to come to a material which not only expresses the above mentioned characteristics with respect to biocompatibility and adjustable biodegradability but also fulfils the requirements with respect to the application of the material in for instance medical devices.

It is furthermore a goal of the invention to provide a material which may serve as a matrix for controlled release of (bio)active substances, such as drugs or growth factors.

The inventors have now found a polymeric material in which certain components have been incorporated to provide these desired characteristics and to fulfil the above requirements. This polymeric material is based on a combination of a polyether component, a short chain diol and a mixture of aromatic and non-aromatic dicarboxylic acids or derivatives thereof.

The invention is accordingly directed to a medical device comprising a random copolymer comprising monomeric units

A: 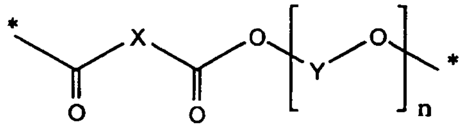 ;

B: 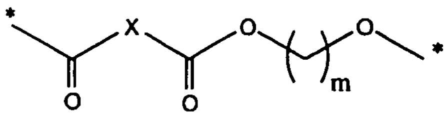 ;

C: 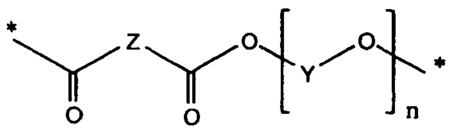 ;

and

D: 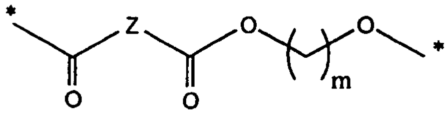 ,

wherein

* represents a linkage to another monomeric unit;
X represents a moiety containing an aromatic group;
Y is an alkylene moiety;
Z represents an alkylene or alkenylene moiety;
n is an integer in the range of 1- 250, preferably 8 - 100; and
m is an integer in the range of 2 - 16.

A polymeric material according to the invention has been found to possess excellent characteristics with regard to biodegradability and biocompatibility. By varying the ratio between the different components of the multiblock copolymer, the biodegradability profile, as well as the mechanical properties, of the polymeric material may conveniently be adjusted. Advantageously, it has been found that under certain conditions the degradation product of the polyester component, the dicarboxylic acid, may catalyse the degradation of the other components of the present polymeric material.

In the context of the present invention, the term biocompatible is intended to refer to materials which may be

incorporated into a human or animal body, *e.g.* in the form of a medical implant, substantially without unacceptable responses of the human or animal. The term biodegradable refers to materials which, after a certain period of time, are broken down in a biological environment. Preferably, the rate of breakdown is chosen similar or identical to the rate at which the body generates autogenous tissue to replace an implant of which the biodegradable material is manufactured.

The term random copolymer is intended to reflect that a polymeric material of the invention comprises monomeric units A, B, C, and D in any order an in any ratio. The ratio of the number a of monomeric units A to the number b of monomeric units B (a/b) will typically be the same as the ratio of the number c of monomeric units C to the number d of monomeric units D (c/d). It is preferred that a copolymer according to the invention does not contain any other monomeric units than the units A, B, C, and/or D.

As such, the polymeric material comprises both hard and soft fragments. The weight fraction of soft fragments may be defined as x and be determined by the combined weight fraction of the monomeric units A and C in the polymeric material. The weight fraction of hard fragments is then defined as 1-x and is determined by the combined weight fraction of the monomeric units B and D in the polymeric material. The weight fraction of soft segments x preferably is in the range of 0.1 to 1, more preferably 0.4 to 1, yet more preferably 0.5 to 0.9. Further, the fraction of the sum of the number of monomeric units C and the number of monomeric units D (c + d) is preferably from 0.01 to 1, more preferably from 0.05 to 0.99.

As mentioned, X is a moiety comprising an aromatic group. The aromatic group may in principle be any aromatic group such as a phenyl, naphtyl, or cyclopentadienyl group. Preferably, the aromatic group is a phenyl group. It will be understood that the aromatic group may carry one or more substituents, such as chloro, bromo, iodo, fluoro, nitro, methoxy groups or alkyl groups containing 1-3 carbon atoms. It is further possible that moiety X comprises other atomic groups, such as methylene groups. Examples of such possibilities include $-(CH_2)_p$-aromatic group-$(CH_2)_q$, wherein p and q are independently chosen from the group of integers in the range of 0-3. Preferably, both p and q are 0.

Moiety Y is an alkylene group. The terms alkylene and polyalkylene, as used herein, generally refer to any isomeric structure, i.e. propylene comprises both 1,2-propylene and 1,3-propylene, butylene comprises 1,2-butylene, 1,3-butylene, 2,3-butylene, 1,2-isobutylene, 1,3-isobutylene and 1,4-isobutylene (tetramethylene) and similarly for higher alkylene homologues. Preferably, this alkylene group is chosen from the group of ethylene, propylene, butylene and combinations thereof. Together with the attached oxygen, moiety Y forms a repeating unit that occurs n times in the monomeric units A and C. This repeating unit is preferably derived from a poly(alkylene glycol). The poly(alkylene glycol) is preferably chosen from the group of poly(ethylene glycol), poly(propylene glycol), and poly(butylene glycol) and copolymers thereof, such as poloxamers. A highly preferred poly(alkylene glycol) is poly(ethylene glycol). The poly (alkylene glycol) may have a weight average molecular weight of about 200 to about 10,000 g/mol. Preferably, the poly (alkylene glycol) has a weight average molecular weight of 300 to 4,000 g/mol. Accordingly, integer n is preferably chosen in the range of 1-250, more preferably 8-100.

A weight average molecular weight, as referred to herein, may suitably be determined by gel permeation chromatography (GPC). This technique, which is known per se, may for instance be performed using chloroform, hexafluoro isopropanol or m-cresol as a solvent and polystyrene or poly(methyl methacrylate) as external standard. Alternatively, a measure for the weight average molecular weight may be obtained by using viscometry (see NEN-EN-ISO 1628-1). This technique may for instance be performed at 25°C using chloroform, hexafluoro isopropanol or a mixture of both as a solvent. Preferably, the intrinsic viscosity of the copolymer lies between 0.2 and 2.0 dL/g. The more preferred ranges for the weight average molecular weight measured by GPC mentioned above can also be expressed in terms of the intrinsic viscosity, when the appropriate physical data for the polymers are known.

Moiety Z is an alkylene or alkenylene group. Like the term alkylene group, the term alkenylene group is intended to refer to any isomeric structure. Thus, for example, a propenylene group can be a 1,2-propenylene group, a 2,3-propenylene group, or an isopropenylene group. Typically, moiety Z, together with the two attached carbonyl groups will be derived from a dicarboxylic acid or a derivative thereof. Preferably, the dicarboxylic acid is an alkanedioic acid having from 4 to 18 carbon atoms. It is further preferred that the dicarboxylic acid is an unbranched dicarboxylic acid, as it has been found that this leads to a copolymer having superior processability, wherein a biologically active agent can be incorporated in a convenient manner. Particularly preferred are dicarboxylic acids chosen from the group of maleic acid, succinic acid, adipic acid, sebacic acid, glutaric acid, and suberic acid. Thus, Z is preferably chosen from the group of ethenylene, propylene, butylenes, hexylene, and octylene. In addition, it has been found that a copolymer having increased swelling behaviour is obtained when the amount of (aliphatic) dicarboxylic acid used to prepare the copolymer is increased.

Instead of using an actual dicarboxylic acid, it is also possible to use a derivative of a dicarboxylic acid which will be incorporated into the copolymer structure by way of the same carboxylic functionalities. Examples of such derivatives can easily be conceived by the skilled person and include anhydrides, mono- and diesters, mono- and diacid chlorides, and the like. In case a mono- or diester of the dicarboxylic acid is used, the carboxylic acid group is preferably esterified to an alkyl group having from 1 to 4 carbon atoms. Preferred derivatives are dimethyl esters.

Integer m is chosen in the range of 2-16. The 2-16 repeating methylene groups are preferably derived from a

diol. In a preferred embodiment, the diol is chosen from the group of dihydroxy ethane, dihydroxy propane or dihydroxy butane. A highly preferred short chain diol is dihydroxy butane.

The weight average molecular weight of a random copolymer according to the invention preferably lies between 10, 000 and 500, 000 g/mol, more preferably between 40,000 and 300,000 g/mol.

The invention further relates to a process for preparing a copolymer as described above comprising the steps of

a) reacting an aromatic dicarboxylic acid or a derivative thereof, a non-aromatic dicarboxylic acid or a derivative thereof with a poly(alkylene glycol) and an alkanediol; and
b) carrying out a polycondensation under removal of the alkanediol.

The aromatic dicarboxylic acid or derivative thereof is chosen such that it gives moiety X described above. A preferred example of the aromatic dicarboxylic acid is terephthalic acid or a derivative thereof. Examples of such derivatives can easily be conceived by the skilled person and include anhydrides, mono- and diesters, mono- and diacid chlorides, and the like. In case a mono- or diester of the dicarboxylic acid is used, the carboxylic acid group is preferably esterified to an alkyl group having from 1 to 4 carbon atoms. Preferred derivatives are dimethyl esters.

The non-aromatic dicarboxylic acid or derivative thereof, as discussed above, is chosen such that it gives moiety Z in the copolymer. Preferably, the non-aromatic is chosen from the group of maleic acid, succinic acid, adipic acid, sebacic acid, glutaric acid, and suberic acid. Examples of suitable derivatives can easily be conceived by the skilled person and include anhydrides, mono- and diesters, mono- and diacid chlorides, and the like. In case a mono- or diester of the dicarboxylic acid is used, the carboxylic acid group is preferably esterified to an alkyl group having from 1 to 4 carbon atoms. Preferred derivatives are dimethyl esters.

The poly(alkylene glycol) is chosen such that it gives moiety Y in the copolymer. The poly(alkylene glycol) is preferably chosen from the group of poly(ethylene glycol), poly(propylene glycol), and poly(butylene glycol) and copolymers thereof, such as poloxamers. A highly preferred poly(alkylene glycol) is poly(ethylene glycol). The poly(alkylene glycol) may have a weight average molecular weight of about 200 to about 10,000 g/mol. Preferably, the poly(alkylene glycol) has a weight average molecular weight of 300 to 4,000 g/mol.

The alkanediol is preferably a short chain diol and is chosen such that it gives the group carrying the index m in the formulas above. In a preferred embodiment, the diol is chosen from the group of dihydroxy ethane, dihydroxy propane or dihydroxy butane. A highly preferred short chain diol is dihydroxy butane.

A preferred preparation of a multiblock copolymer according to the invention will now be explained by way of example for a multiblock copolymer synthesized from poly(ethylene glycol), butanediol, dimethyl terephthalate, and dimethyl succinate. Based on this description, the skilled person will be able to prepare any desired multiblock copolymer within the above described class.

A typical poly(ether ester) multiblock copolymer may be synthesized from a mixture of dimethyl terephthalate, an alkanediol such as 1,4-butanediol (in excess), poly(ethylene glycol), dimethyl succinate, an antioxidant and a catalyst. An example of a suitable catalyst is tetrabutyloxy titanium. The mixture is placed in a reaction vessel and preferably heated to at least about 160°C, and methanol is preferably distilled as transesterification proceeds.

After transesterification, the temperature is preferably raised slowly to about 245°C, and a vacuum (finally less than 0.1 mbar) is preferably achieved. Excess alkanediol may be distilled off and the objective multiblock copolymer is formed in a polycondensation step.

Due to its advantageous properties a polymeric material according to the invention can be used for the manufacture of various medical devices, such as scaffolds for artificial skin, bone implants, cement restrictors, or for tissue engineering cartilage or muscle.

The invention therefore also encompasses a medical device comprising a polymeric material as described above. Preferably, the medical device can be used for tissue repair or controlled drug release. Preferred forms of the medical device include a scaffold for engineering tissue *in vitro,* a matrix for controlled drug release, a substitute for tissue repair, a suture, a bone screw, a suture anchor, a tapered or non-tapered pin, or a staple.

It is noted that based on the above description of a polymeric material according to the invention, its composition and its preparation, the skilled person can adjust the properties of the material to suit his particular needs in the context of a specific application or device. For instance, a more flexible material may be desired for scaffolds for tissue engineering skin or for sutures, which property is positively affected by increasing the amount of the poly(alkylene glycol) component in the multiblock copolymer. For other purposes such as controlled release of (bio)active agents, an even faster degradation profile is desired in which case higher amounts of the non-aromatic dicarboxylic acid or derivative thereof may be incorporated. In this regard, it is to be noted that a given wt.% of poly(alkylene glycol) component in the multiblock copolymer can be incorporated in two different ways. One possibility is to use a lower number of larger blocks, i.e. poly (alkylene glycol) of a higher weight average molecular weight; another is to incorporate more blocks of a lower weight average molecular weight.

In order to obtain a medical device suitable for application the polymeric material is prepared by conventional

processing step for such as extrusion, injection-molding, blow-molding, solution molding and other techniques for the shaping and processing of polymeric materials.

To further enhance the performance of the medical device according to the invention it is possible to include pharmaceuticals or pharmaceutically active components in the polymeric composition. The device can be used for the controlled release of medicaments. This can be performed in conjunction with the other functions of the polymeric composition of the present invention, but it may also be the sole purpose of this specific embodiment.

Besides as a device, the polymer of the present invention can also be used to prepare controlled release formulations for the release of biologically active agents, including protein and peptides. Such delivery systems may be formulated into microspheres, injectable gels, sheets etc., by methods known to those skilled in the art.

The term "biologically active agent", as used herein, means an agent which provides a therapeutic or prophylactic effect. Such agents include, but are not limited to, antimicrobial agents (including antibacterial and antifungal agents), anti-viral agents, anti-tumor agents, hormones immunogenic agents, growth factors, lipids, and lipopolysaccharides.

The above mentioned biologically active agents that can be incorporated into the polymeric material may vary widely in nature; in principle any type of additive may be incorporated. As the polymeric material is biodegradable *in vivo,* and allows diffusion of molecules, the additives will be released to the surroundings of the material in a controlled manner. These additives may be added to the solution in amounts ranging from 0 to 50 wt.%, preferably from 1 to 20 wt.%.

Biologically active agents which may be incorporated include, but are not limited to, non-peptide, non-protein small-sized drugs. They have a molecular weight which in general is less than 1500, and in particular less than 500. A second important group of biologically active agents are biologically active peptides and proteins.

A biologically active agent may be incorporated into the polymeric material by dissolving it in a solution of the polymeric material. Suitable solvents are chloroform, dichloromethane, N-methyl-2-pyrrolidone, dimethyl sulfoxide, acetone, hexafluoroisopropanol and the like. The selection of a suitable solvent will be dependent on the composition of the chosen copolymer. Hence, a homogeneous solution is formed or a suspension is formed by dispersion. Alternatively, a solution of a biologically active agent may be mixed with the copolymer solution to form a homogeneous mixture, or an emulsion. Also, a biologically agent can be incorporated by physically mixing with the copolymer, for example by extrusion. Since in the latter case heat is applied, care must be taken not to harm the stability and/or activity of the biologically active agent.

If it is desired that a porous material is formed, a pore-forming agent can be included in the solution or suspension of the copolymer. Pore-forming agents may include organic solvents, water, salts (sodium chloride, sodium citrate, and the like), sugars and water-soluble synthetic polymers. Using such pore-forming agents, pores can be created by leaching-out of the agent, or by phase separation.

The invention will now be further elucidated by the following, non-restrictive examples.

Example 1.

The poly(ether ester) multiblock copolymer described in the following example is composed from poly(ethylene gycol), butanediol, dimethyl terephthalate as the aromatic dicarboxylic acid derivative and dimethyl succinate as non-aromatic dicarboxylic acid. A polymer (1-A) that contains approximately 60% by weight of poly(ethylene glycol) and terephthalate and succinate in a 50/50 molar ratio is prepared by placing the following materials in a reactor suitable to perform both atmospheric distillations and distillations under reduced pressure:

Table 1. Raw materials used to prepare poly(ether ester) 1-A

| Raw materials | 1 kg reactor (g) |
|---|---|
| Poly (ethylene glycol) (MW = 1000 g/mol) | 531 |
| Dimethyl succinate | 172 |
| Dimethyl therephthalate | 228 |
| 1,4-butanediol | 409 |
| $\alpha$-tocopherol | 6.3 |
| Tetrabutyl ortho titanate | 0.91 |

The reactor is equipped with a mechanical stirrer with torque read-out, a nitrogen inlet tube, a Pt100 temperature sensor connected to a digital read-out device and a condensor, which can be heated by a thermostatic water bath. The reactor is heated by a thermostatic oil bath.

The transesterfication reaction begins at 140 to 145°C. Methanol is distilled off for approximately one hour. After this the temperature is slowly increased to 240°C. As the temperature of the reaction mixture reaches 230 to 240°C,

the pressure is gradually reduced to 0,5-1,5 mbar in approximately 30 minutes. During 3-4 hours, until the reaction mixture has reached the desired viscosity, the condensation product 1,4-butanediol is distilled off. The polymer is then extruded and quenched in cold water follow by drying and grinding.

The intrinsic viscosity of the product measured in chloroform at 25°C is 0,990 dl/g. H-NMR measurements were used to calculate the polyether wt.% and the diacid ratio (T/S). For example 1-A the poly(ethylene glycol) content was 58.3 wt.%. The diacid ratio (T/S) is 55/45 mole%.

Example 2.

For comparison, a multiblock copolymer (1-B) was synthesized that contains approximately the same polyether content as 1-A. The diacid used was only succinate, no aromatic diacid was incorporated. It is prepared in the same way as described in example 1, by placing the following materials in a reactor:

Table 2. Raw materials used to prepare poly(ether ester) 1-B

| Raw materials | 1 kg reactor (g) |
|---|---|
| Poly (ethylene glycol) (MW = 1000 g/mol) | 563 |
| Dimethyl succinate | 414 |
| 1,4-butanediol | 511 |
| $\alpha$-tocopherol | 6.3 |
| Tetrabutyl ortho titanate | 0.98 |

The intrinsic viscosity of the product measured in chloroform at 25 °C is 1,166 dl/g. H-NMR measurements showed that 63.5 w/w% poly(ethylene glycol) was incorporated.

Example 3.

A 10% by weight solution of the polymers described in examples 1 and 2 were used to cast films to be used for in-vitro degradation studies. Dry films (approximately 0.5 gram, 50-100 $\mu$m thickness) were immersed in 50 ml phosphate buffered saline (PBS, pH 7.4, containing 1.06 mM $KH_2PO_4$, 155.17 mM NaCl, and 2.96 mM $Na_2HPO_4$•7 $H_2O$) at 37°C in a shaking bath for 1, 2, 4 and 8 weeks. Each week, the buffer was refreshed. The films were freeze-dried and subsequently analysed by Gel Permeation Chromatography (GPC). Samples were eluted in 0.02M sodium-trifluoroacetate ($NaF_3Ac$) in hexafluoroisopropanol (HFIP) through a Polymer Labs HFIP gel guard column (50 x 7.5 mm) and two PL HFIP gel analytical columns (300 x 7.5 mm). Flow rate was 1 ml/min and a Refraction Index (RI) detector was used. Column temperature was 40°C and sample concentration was 20 mg/ml. The molecular weights (Mn and Mw) were determined relative to polymethylmethacrylate (PMMA) standards.

As a reference the degradation of a poly(ether ester) multiblock copolymer containing approximately the same polyether content as 1-A is included (data from US patent 5,980,948). The diacid used in the reference polymer was only terephthalate, no non-aromatic diacid was incorporated. It is prepared in the same way as described in example 1.

Table 3. Molecular weight of poly(ether ester) multiblock copolymers after degradation in phosphate buffer saline (pH = 7.4).

| Time [weeks] | Polymer 1-B | | Polymer 1-A | | Reference | |
|---|---|---|---|---|---|---|
| | Mw [D] | relative [%] | Mw [D] | relative [%] | Mw [D] | relative [%] |
| 0 | 158541 | 100 | 143470 | 100 | 102000 | 100 |
| 1 | 102471 | 65 | 118022 | 82 | 89300 | 87 |
| 2 | 72546 | 46 | 99565 | 69 | 84000 | 82 |
| 4 | 44554 | 28 | 73482 | 51 | 70600 | 69 |
| 8 | 36513 | 23 | 53702 | 37 | 60000 | 59 |

From the results presented in table 3 it is obvious that an increase in the amount of non-aromatic diacids incorporated in the poly(ether ester) multiblock copolymers results in an increased rate of degradation.

Example 4: Protein release from aliphatic/aromatic poly(ether ester) films

I. Introduction

To evaluate PEG(T/S)/PB(T/S) copolymers (herein PEG stands for polyethyelene glycol, PB stands for polybutylene, T stands for terephthalate, and S stands for succinate) as matrix for the controlled release of proteins, the in-vitro release was studied from films. The effect of the matrix composition was studied by varying the PEG molecular weight and the percentage of succinate. In addition, model proteins of different sizes were investigated.

II. Materials and Methods

An overview of the experiments of Example 4 is presented in the attached Appendix 1.

Table 4: materials and apparatus used for the films study

<table>
<tr><th>Description</th><th>Manufacturer</th></tr>
<tr><td>300PEG(T/S)60PB(T/S)40 T/S=50/50</td><td>Iso Tis N.V.</td></tr>
<tr><td>600PEG(T/S)60PB(T/S)40 T/S=50/50</td><td>IsoTis N.V</td></tr>
<tr><td>1000PEG(T/S)60PB(T/S)40 T/S=50/50</td><td>IsoTis N.V.</td></tr>
<tr><td>4000PEG(T/S)60PB(T/S)40 T/S=50/50</td><td>IsoTis N.V.</td></tr>
<tr><td>1000PEG(T/S)60PB(T/S)40 T/S=90/10</td><td>IsoTis N.V.</td></tr>
<tr><td>1000PEGS60PBS40</td><td>IsoTis N.V.</td></tr>
<tr><td>1000PEGT65PBT35</td><td>IsoTis N.V.</td></tr>
<tr><td>Lysozyme</td><td>Sigma, USA</td></tr>
<tr><td>Bovine Serum Albumin</td><td>Sigma, USA</td></tr>
<tr><td>Carbonic Anhydrase</td><td>Sigma, USA</td></tr>
<tr><td>Chloroform</td><td>Fluka chemica, Switzerland</td></tr>
<tr><td>Dichloromethane</td><td>Fluka chemica, Switzerland</td></tr>
<tr><td>Micro BCA</td><td>Omnilabo</td></tr>
<tr><td>Coomassie Plus Protein Assay Reagent Kit</td><td rowspan="6">Omnilabo Eppendorf, Germany</td></tr>
<tr><td>1.5 mL Safe lock tubes</td></tr>
<tr><td>Eppendorf 10-100 µl</td></tr>
<tr><td>Multipipette Epp 30-300 µl</td></tr>
<tr><td>Eppendorf 20-200 µl</td></tr>
<tr><td>Eppendorf 100-1000 µl</td></tr>
<tr><td>Eppendorf 500-5000 µl</td><td></td></tr>
<tr><td>Film applicator</td><td>411/220, Erichsen, Germany</td></tr>
<tr><td>Plates 96 wells Plates 96 wells</td><td>Greiner laboratories, The Netherlands</td></tr>
<tr><td>Ultra Turrax</td><td>T25, IKA labortechnik, The Netherlands</td></tr>
<tr><td>Freeze dryer</td><td>Alpha 1-4, CHRIST, Germany</td></tr>
<tr><td>Water bath</td><td>OLS 200, GRANT laboratories</td></tr>
<tr><td>Spectrophotometer</td><td>EL 312e, BioTek instruments, USA</td></tr>
</table>

*1. Preparation of films*

Films were prepared from a water-in-oil emulsion (w/o). The oil phase was constituted of 1 gram of polymer

dissolved in at least 5 mL of dichloromethane (for the exact amounts, see appendix 1). The water phase consisted of the protein dissolved in PBS (55 mg/mL). The 3 different proteins used were Lysozyme, Carbonic Anhydrase and Bovine Serum Albumin (BSA). 0.6mL of this protein solution was added to the polymer solution and stirred a few seconds with a stirring plate. Then, the mix was put in a 50 mL centrifuge tube and the emulsion was prepared using the Ultra Turrax for 30 seconds at 19000 rpm.

The emulsion was cast using an adjustable film applicator (setting: 700 μm) on a glass plate. After evaporation of the dichloromethane, the films were stripped from the glass plate and dried further in the air for some hours. Subsequently, the films were dried in the freeze-dryer for at least 10 hours.

*2. Release determination*

For each film, three pieces (+/- 1.77 $cm^2$) were weighed and incubated in 1 ml of the release buffer (Phosphate Buffer Saline pH 7.4, PBS) at 37°C in a water bath, under a constant agitation (26/min). At several intervals (depending on the release rate), the release medium was replaced.

To determine the amount of protein that has been released, a spectrophotometer was used: first a calibration curve of different concentrations (from 0 up to 25 μg/ml) in PBS was made, using the Micro BCA protein assay (detection wavelength 570 nm).

This standard curve was used to determine the concentration of protein in the release medium.

*3. Experiments performed*

All the different protein loaded films prepared and characterized during this study are listed in the appendix 1. The T/S ratio was approximately 50/50 mol%, unless stated different.

III. Results and Discussion

*A. Effect of the percentage of succinate in the copolymer composition on the release*

The experiments were performed with different percentages of succinate in the copolymers (T/S ratio) whereas the PEG molecular weight (1000g/mol), the PEG(T/S) weight percentage (60), and the PB(T/S) weight percentage (40) were kept constant. The PEGT/PBT copolymer 1000PEGT65PBT35 was used as a reference, and Lysozyme was chosen as a model protein for this release study.

It was necessary to make a correction for the release curves because some of the films had an apparent release higher than 100%. The correction was made considering the highest release obtained as 100%. This could be due to the difficulty to remove the entire release medium when the buffer is refreshed. Evaporation of water and condensation on the lid can also affect the concentrations.

Graphs 1A and B: Cumulated release (%) of lysozyme from 1000PEG(T/S)60PB(T/S)40 when the percentage of succinate was 10%, 50%, 100% and from the 'reference' 1000PEGT65PBT35.

The copolymer which contains 100% of succinate showed a very fast release of lysozyme (graph 1A): 100% of the protein is released within 20 minutes. When the succinate percentage is only 50%, the release was finished after 5 hours. Two release behaviours are clearly visible on the overall graph of the experiment (graph 1B): in contrast to the fast release from polymers with 50 or 100% succinate, the release from polymers with 0 or 10% succinate continued for more than 30 days. For the compositions containing 50% and 100% of succinate, the fast release is due to the swelling of the copolymers. The more succinate was present, the higher the swelling of the matrix was (see example 6) and consequently, the faster the protein can pass trough it. For the 0 and 10% succinate compositions, the degradation of the matrix is also involved because the release takes more time. Moreover, the degradation of the 0 and 10% succinate compositions have the same profile which explains also the similar profile of their release. The combination of degradation and diffusion explains the zero order profile observed for the 0 and 10% of succinate compositions. For highly swollen matrices, in which diffusion is fast compared to degradation, no effect of polymer degradation can be expected and a first order profile of release is observed.

In conclusion, the release behaviour depends strongly of the percentage of succinate in the copolymer.

*Diffusion Coefficient Determination:*

As the release is very sensitive to the percentage of succinate, the determination of the diffusion coefficient of each copolymer is interesting. The diffusion coefficient expresses the capability of the molecule entrapped to diffuse through the polymer matrix into the release medium. In order to quantify the effect of the percentage of succinate in the copolymer on the release and calculate the diffusion coefficient, the release was plotted as a function of t (time in

seconds). The diffusion coefficients were calculated using equations 2 and 3, after rearranging.

*Equations 2 and 3:*

**If $M_t/M_\infty > 0{,}4$:** $$\frac{M_t}{M_\infty} = 1 - \frac{8}{\pi^2} \times \exp\left(-\frac{\pi^2 Dt}{l^2}\right)$$ **and if $M_t/M_\infty < 0{,}6$:** $$\frac{M_t}{M_\infty} = 4\sqrt{\frac{Dt}{\pi l^2}}$$

(in which $M_t/M_\infty$ is the release, D is the diffusion coefficient, and l is the film thickness).

The diffusion coefficients calculated for the various copolymers compositions are given in the table 5 below. In graph 2, the effect of the percentage of succinate on the diffusion coefficient is shown. Lysozyme loaded films from 1000PEG(T/S)60PB(T/S)40 composition were used.

Table 5

| Succinate (%) in the copolymer | Diffusion Coefficient (D, $cm^2/s$) |
|---|---|
| 0 | $(0.01360.026)x10^{-10}$ |
| 10 | $(0.1660.018)x10^{-10}$ |
| 50 | $(23.863)x10^{-10}$ |
| 100 | $(290626)x10^{-10}$ |

The more succinate is present in the copolymer, the higher the diffusion coefficient is. This is even more visible in graph 2. Therefore, if there is 100% of succinate in the copolymer, the lysozyme can pass through the matrix relatively easy.

For a composition of a PEGT/PBT copolymer which has roughly the same swelling coefficient, succinate copolymers have a higher diffusion coefficient [Bezemer J.M., Protein Release Systems based on Biodegradable Amphiphilic Multiblock copolymers, Thesis University of Twente; 1992 p.65].

*B. Effect of the PEG segment length of the copolymer on the release*

The experiments were performed with four different PEG molecular weights copolymers, but all had approximately the same T/S ratio: around 50/50 mol/mol. Three proteins of different sizes were used to characterize the release properties of the copolymers:

- Lysozyme: 14,5 kD
- Carbonic Anhydrase: 29 kD
- BSA (Bovine Serum Albumin): 67 kD.

In graph 4, the release of lysozyme is given.

The release of lysozyme was complete within only 5 hours for the 1000PEG(T/S)60PB(T/S)40 (graph 3), and within 1 hour for the 4000PEG(T/S)60PB(T/S)40. The 600PEG(T/S)60PB(T/S)40 needs 10 days to release all the Lysozyme entrapped, whereas only 5% of the protein is released in 25 days from the 300PEG(T/S). The lysozyme must be retained inside of the matrix of the 300PEG(T/S). As the release is fast for the copolymers containing PEG segments of 1000 and 4000 g/mol, the release is mostly determined by the matrix swelling instead of degradation. The 4000PEG showed the fastest release because it swells more than the other compositions (appendix 1), resulting in the highest diffusion coefficient.

BSA is the biggest protein (67kD) used for this release study (graph 4). The 1000PEG polymer has a complete release in 80 days and the 4000PEG in 100 days, whereas the BSA release from the 600PEG polymer is not finished yet at day 125.

The large size of the protein induces a lag-time at the beginning: there is no initial diffusion out of the matrix. If the release was mainly determined by the swelling of the matrix, an increasing release rate would be expected with

increasing PEG segment length. Surprisingly, the release from the 1000 composition is the fastest and is therefore linked to the degradation scheme. Around 40-50 days, the matrix is more open for protein diffusion, due to degradation. Moreover, the release of the 4000 composition is faster than the 600 composition whereas the degradation profiles were the same. This is due to a combination of swelling and degradation effects.

*C. Effect of the nature/size of the protein on the release*

The experiments were performed with the same 3 PEG lengths as previously (600, 1000 and 4000) and the same percentage of succinate (50%). By the same way, Lysozyme, Carbonic Anhydrase and BSA proteins were also used.

As an example, the releases of BSA, Lysozyme and Carbonic Anhydrase from the 1000PEG(T/S) composition are given below in graph 5.

Two release profiles can be observed in graph 5. Smaller proteins like Lysozyme or Carbonic Anhydrase showed a short-term release (within hours/days) more due to diffusion through the matrix. First, as BSA is too big, there is nearly no diffusion. Then, the BSA diffusion increases because of the degradation. In conclusion, when the protein size increases, the release rate decreases.

IV. Conclusion

New succinate containing copolymers were evaluated for release purposes because the degradation rate of PEGT/PBT copolymers was too slow for some applications. It was shown that protein release was faster for succinate containing polymers than for the aromatic poly(ether ester)s. Lysozyme was released from the 1000PEG(T/S)60PB(T/S) 40 (50% succinate) within 5 hour, whereas for the comparable PEGT/PBT composition it takes 30 days. When the PEG molecular weight increased, the release was faster. By the same way, when the percentage of succinate was higher, the release was also faster. Two release profiles were observed. For small proteins (lysozyme, carbonic anhydrase), the release was fast through the swollen matrix and a first order release was obtained. For larger protein (BSA), the release was correlated to the degradation behaviour of the copolymer and a delayed release profile was observed.

<u>Example 5: In vivo degradation</u>

Porous discs (d=16 mm) were prepared using a solvent casting/salt leaching process. Gamma irradiated samples were implanted subcutaneous on the back along the dorso-medial line of large Wistar rats. Samples were explanted after 2, 8 and 15 weeks. GPC analysis on the copolymers was performed after extraction from the tissue using chloroform.

For this investigation, four copolymer compositions have been used with a fixed PEG Mw (1000D) and PEG (T/S)/PB(T/S) ratio (65/35) and variable succinate substitution ratio. In the results, for instance 10% S means that the ratio of terephthalate to succinate is 90 / 10.

The results of the in vivo degradation (molecular weight decrease) are presented in graph 6. For comparison, the in vitro molecular weight loss is presented in graph 7.

The degradation in-vivo seems to go faster than in-vitro. In contrast to the in-vitro degradation, the 10% substitution degrades clearly faster than the 0% substituted copolymer. Comparable trends can be observed for the other copolymers, only at a faster rate.

<u>Example 6: Swelling behaviour</u>

Four copolymer compositions have been synthesized as described in example 1. The copolymer had a fixed PEG Mw (1000D) and PEG(T/S)/PB(T/S) ratio (65/35) and variable succinate substitution ratio. A substitution of for example 10% indicates that 10 mol % of the diacid groups consist of succinate, the remaining 90 mole % is terephthalate.

The swelling of the films is determined by weighing them before and after incubation in the release buffer for three days. The equation used to calculate the equilibrium volume-swelling ratio is shown below in the equation 1:

$$Q = 1 + \frac{1.2 \times (M_{swollen} - M_{dry})}{M_{dry}}$$; In which 1.2 is the copolymer density.

*Equation 1: equilibrium swelling ratio.*

Results

Table 6 below clearly shows that an increase in substitution of the terephthalic groups by succinate groups increases the swelling ratio.

Table 6

| Substitution of terephthalate by succinate (Mol %) | Swelling |
|---|---|
| 0 | 1.65 |
| 10 | 1.76 |
| 45 | 2.22 |
| 100 | 2.52 |

| Sample Code | Copolymer Composition | T/S (mol/mol) | $CH_2Cl_2$ (mL) | Protein (55mg/mL) | Time to reach max. release (days) | Swelling | Thickness (μm) |
|---|---|---|---|---|---|---|---|
| A | 600PEG(T/S)60PB (T/S)40 | 50/50 | 5 | BSA | 103 | 1.860.021 | 92 |
| B | 1000PEG(T/S)60PB (T/S)40 | 50/50 | 5 | BSA | 89 | 2.460.040 | 95 |
| C | 4000PEG(T/S)60PB (T/S)40 | 50/50 | 5 | BSA | 103 | 3.360.024 | 130 |
| D | 600PEG(T/S)60PB (T/S)40 | 50/50 | 5 | Lysozyme | 12 | 1.760.011 | 111 |
| E | 1000PEG(T/S)60PB (T/S)40 | 50/50 | 5 | Lysozyme | 0.208 | 2.260.068 | 105 |
| F | 4000PEG(T/S)60PB (T/S)40 | 50/50 | 5 | Lysozyme | 0.083 | 3.160.021 | 132 |
| G | 300PEG(T/S)60PB (T/S)40 | 50/50 | 5 | Lysozyme | 17 | 1.260.029 | 92 |
| H | 2000PEGT80PBT20 | 100/0 | 5 | Carbonic Anhydrase | 1 | 3.360.058 | 159 |
| I | 600PEG(T/S)60PB (T/S)40 | 50/50 | 5 | Carbonic Anhydrase | 0.2 | 1.760.014 | 103 |
| J | 1000PEG(T/S)60PB (T/S)40 | 50/50 | 5 | Carbonic Anhydrase | 1 | 2.360.080 | 91 |
| | 4000PEG(T/S)60PB (T/S)40 | 50/50 | 5 | Carbonic Anhydrase | | 3.260.033 | 154 |
| | 1000PEG(T/S)60PB (T/S)40 | 50/50 | 8 | Carbonic Anhydrase | | 2.260.08 | 78 |
| | 4000PEG(T/S)60PB (T/S)40 | 50/50 | 8 | Carbonic Anhydrase | 5.5 | 3.060.10 | 99 |
| | 1000PEGT65PBT35 | 100/0 | 7 | Lysozyme | 32 | 1.960.052 | 94 |
| | 1000PEGS60PBS40 | 0/100 | 5 | Lysozyme | 0.208 | 2.860.038 | 112 |
| | 1000PEG(T/S)60PB (T/S)40 | 90/10 | 5 | Lysozyme | 32 | 2.060.006 | 116 |

**Appendix 1**: List of the experiments performed in Example 4

**Claims**

1. Medical device comprising a random copolymer, which copolymer comprises monomeric units

A: 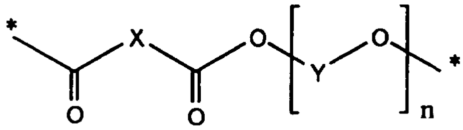 ;

B: 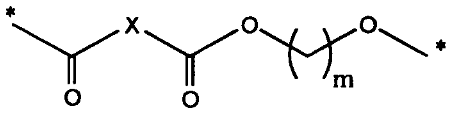 ;

C: 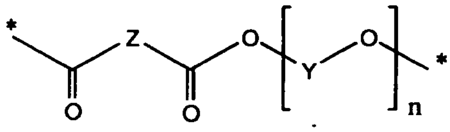 ;

and

D: 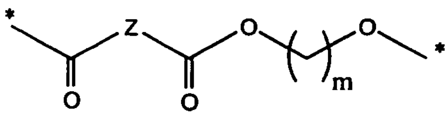 ,

wherein

* represents a linkage to another monomeric unit;
X represents a moiety containing an aromatic group;
Y is an alkylene moiety;
Z represents an alkylene or alkenylene moiety;
n is an integer in the range of 1- 250, preferably 8 - 100; and
m is an integer in the range of 2 - 16.

2. Medical device according to claim 1, wherein the combined weight fraction of the monomeric units A and C of the copolymer lies in the range of 0.1 to 1.

3. Medical device according to claim 1 or 2, wherein the combined weight fraction of the sum of the number of monomeric units C and the number of monomeric units D of the copolymer is from 0.01 to 1, preferably from 0.05 to 0.99.

4. Medical device according to any of the preceding claims, wherein X in the copolymer is a phenyl group.

5. Medical device according to any of the preceding claims, wherein Y in the copolymer is chosen from the group of ethylene, propylene, butylene and combinations thereof.

**6.** Medical device according to any of the preceding claims, wherein Z in the copolymer is chosen from the group of ethenylene, propylene, butylenes, hexylene, and octylene.

**7.** Medical device according to any of the preceding claims, wherein the copolymer has a weight average molecular weight of from 10,000 to 500,000 g/mol, preferably from 40,000 to 300,000 g/mol.

**8.** Medical device according to any of the claims 1-7, being a scaffold for engineering tissue in vitro, a matrix for controlled drug release, a substitute for tissue repair, a suture, a bone screw, a suture anchor, a tapered or non-tapered pin, or a staple.

**9.** Use of a copolymer comprising monomeric units

A: 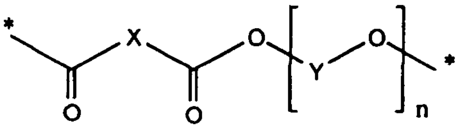 ;

B: 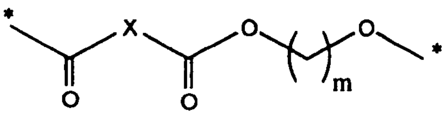 ;

C: 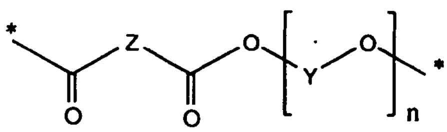 ;

and

D: 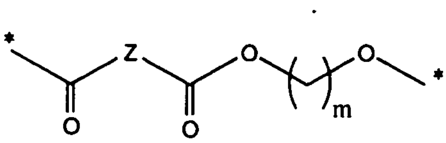 ,

wherein

* represents a linkage to another monomeric unit;
X represents a moiety containing an aromatic group;
Y is an alkylene moiety;
Z represents an alkylene or alkenylene moiety;
n is an integer in the range of 1- 250, preferably 8 - 100; and
m is an integer in the range of 2 - 16,
for the manufacture of a medicament for tissue repair or controlled drug release.

**Patentansprüche**

**1.** Medizinische Vorrichtung, umfassend ein statistisches Copolymer, wobei das Copolymer die folgenden Monomereinheiten umfasst:

A: 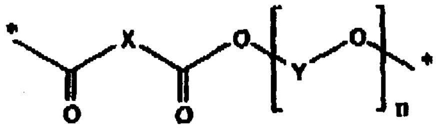 ;

B: 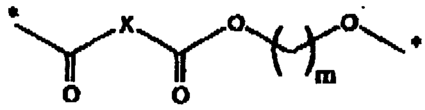 ;

C: 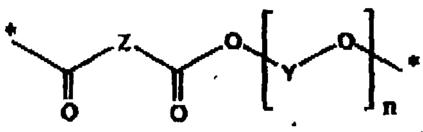 ;

und

D: 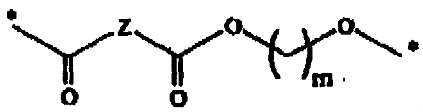 ,

wobei

* eine Verknüpfung zu einer anderen Monomereinheit darstellt;
X eine Einheit darstellt, die einen aromatischen Rest enthält;
Y eine Alkyleneinheit ist;
Z eine Alkylen- oder Alkenyleneinheit darstellt;
n eine ganze Zahl im Bereich von 1 bis 250, bevorzugt 8 bis 100, ist; und
m eine ganze Zahl im Bereich von 2 bis 16 ist.

**2.** Medizinische Vorrichtung gemäß Anspruch 1, wobei die kombinierte Gewichtsfraktion der Monomereinheiten A und C des Copolymers im Bereich von 0,1 bis 1 liegt.

**3.** Medizinische Vorrichtung gemäß Anspruch 1 oder 2, wobei die kombinierte Gewichtsfraktion der Summe der Anzahl der Monomereinheiten C und der Anzahl der Monomereinheiten D des Copolymers 0,01 bis 1, bevorzugt 0,05 bis 0,99, ist.

**4.** Medizinische Vorrichtung gemäß einem der vorhergehenden Ansprüche, wobei X in dem Copolymer eine Phenylgruppe ist.

**5.** Medizinische Vorrichtung gemäß einem der vorhergehenden Ansprüche, wobei Y in dem Copolymer aus der Gruppe Ethylen, Propylen, Butylen und Kombinationen davon ausgewählt ist.

**6.** Medizinische Vorrichtung gemäß einem der vorhergehenden Ansprüche, wobei Z in dem Copolymer aus der Gruppe Ethenylen, Propylen, Butylene, Hexylen und Octylen ausgewählt ist.

**7.** Medizinische Vorrichtung gemäß einem der vorhergehenden Ansprüche, wobei das Copolymer ein Gewichtsmittel des Molekulargewichts von 10.000 bis 500.000 g/Mol, bevorzugt 40.000 bis 300.000 g/Mol, aufweist.

**8.** Medizinische Vorrichtung gemäß einem der Ansprüche 1 bis 7, welche ein Gerüst für Tissue-engineering *in vitro,* eine Matrix für kontrollierte Arzneistofffreisetzung, ein Ersatz für Gewebewiederherstellung, ein Nahtmaterial, eine

Knochenschraube, eine Nahtmatenalbeiestigung, eine sich verjüngende oder sich nicht verjüngende Nadel oder eine Klammer ist.

**9.** Verwendung eines Polymers, umfassend die folgenden Monomereinheiten:

A: 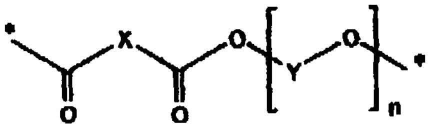 ;

B: 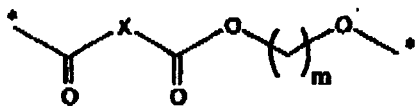 ;

C: 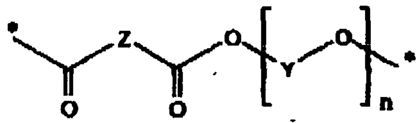 ;

und

D: 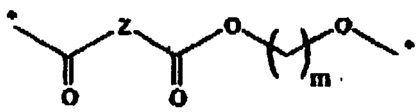 ,

wobei

* eine Verknüpfung zu einer anderen Monomereinheit darstellt;
X eine Einheit darstellt, die einen aromatischen Rest enthält;
Y eine Alkyleneinheit ist;
Z eine Alkylen- oder Alkenyleneinheit darstellt;
n eine ganze Zahl im Bereich von 1 bis 250, bevorzugt 8 bis 100, ist; und
m eine ganze Zahl im Bereich von 2 bis 16 ist,
zur Herstellung eines Medikaments für Gewebewiederherstellung oder kontrollierte Armeistofffreisetzung.

**Revendications**

**1.** Dispositif médical comprenant un copolymère statistique, lequel copolymère comprend des unités monomères :

A: 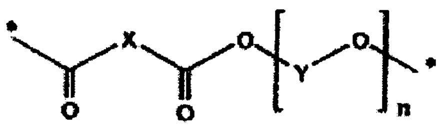 ;

B: 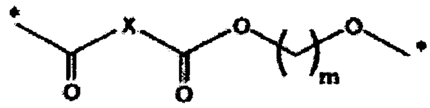 ;

C: 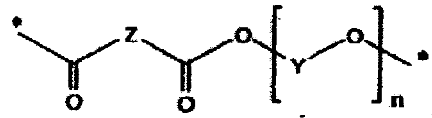 ;

D: 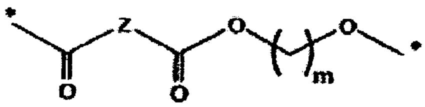 ,

dans lesquelles :

* représente une liaison à une autre unité monomère ;
X représente une entité contenant un groupe aromatique ;
Y est un groupe alkylène ;
Z représente un groupe alkylène ou alcénylène ;
n est un entier compris dans la gamme de 1 à 250, de préférence de 8 à 100 ; et
m est un entier compris dans la gamme de 2 à 16.

2. Dispositif médical selon la revendication 1, dans lequel la fraction pondérale combinée des unités monomères A et C du copolymère est comprise dans la gamme de 0,1 à 1.

3. Dispositif médical selon la revendication 1 ou 2, dans lequel la fraction pondérale combinée de la somme du nombre d'unités monomères C et du nombre d'unités monomères D du copolymère est comprise entre 0,01 et 1, de préférence entre 0,05 et 0,99.

4. Dispositif médical selon l'une quelconque des revendications précédentes, dans lequel X dans le copolymère est un groupe phényle.

5. Dispositif médical selon l'une quelconque des revendications précédentes, dans lequel Y dans le copolymère est choisi dans le groupe comprenant l'éthylène, le propylène, le butylène et leurs combinaisons.

6. Dispositif médical selon l'une quelconque des revendications précédentes, dans lequel Z dans le copolymère est choisi dans le groupe comprenant l'éthénylène, le propylène, les butylènes, l'hexylène et l'octylène.

7. Dispositif médical selon l'une quelconque des revendications précédentes, dans lequel le copolymère a un poids moléculaire moyen en poids de 10.000 à 500.000 g/mole, de préférence de 40.000 à 300.000 g/mole.

8. Dispositif médical selon l'une quelconque des revendications 1 à 7, qui est une ossature de construction de tissu *in vitro,* une matrice pour la libération contrôlée de médicament, un substitut pour la réparation de tissu, une suture, une vis pour os, une ancre de suture, une broche effilée ou non effilée, ou une agrafe.

9. Utilisation d'un copolymère comprenant les unités monomères :

A: 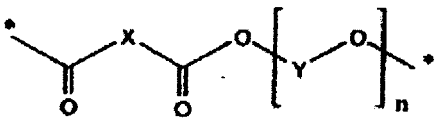 ;

B: 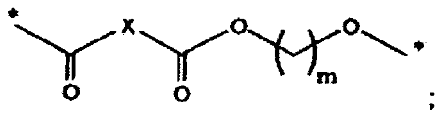 ;

C: 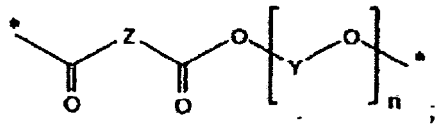 ;

D: 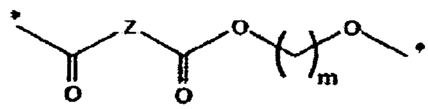 ,

dans lesquelles :

* représente une liaison à une autre unité monomère ;
X représente une entité contenant un groupe aromatique ;
Y est un groupe alkylène ;
Z représente un groupe alkylène ou alcénylène ;
n est un entier compris dans la gamme de 1 à 250, de préférence de 8 à 100 ; et
m est un entier compris dans la gamme de 2 à 16,
pour la fabrication d'un médicament destiné à la réparation de tissu ou à la libération contrôlée de principe actif.

Fig.1

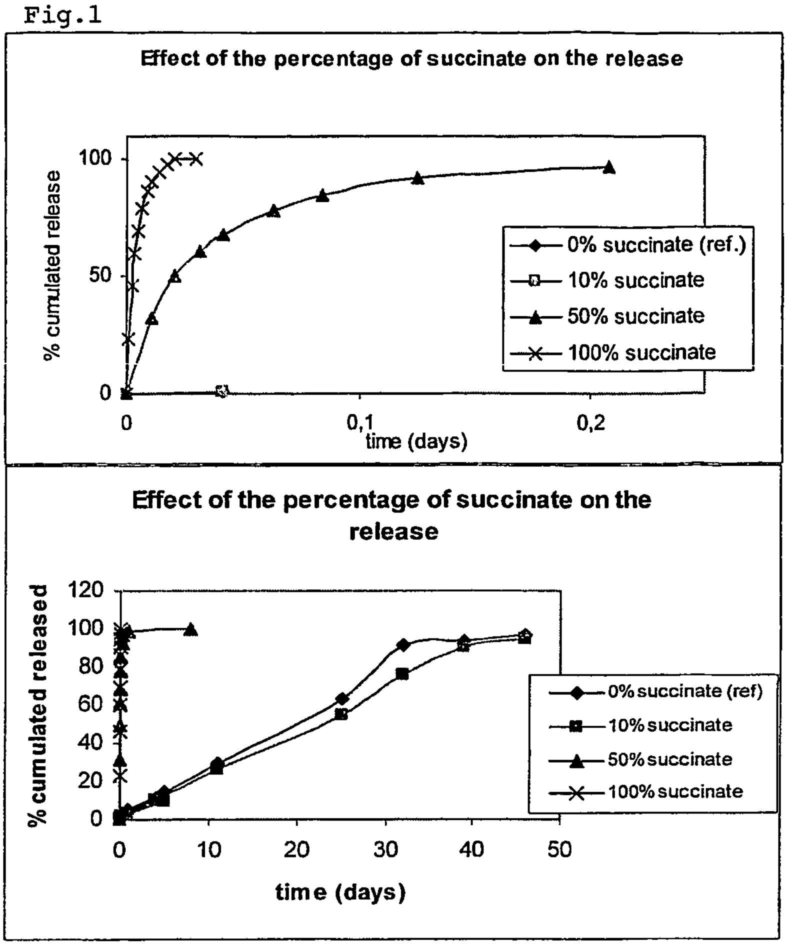


Graphs 1A (initial) and B (long-term): Cumulated release (%) of lysozyme from 1000PEG(T/S)60PB(T/S)40 when the percentage of succinate was 10%, 50%, 100% and from the 'reference' 1000PEGT65PBT35.

Fig.2

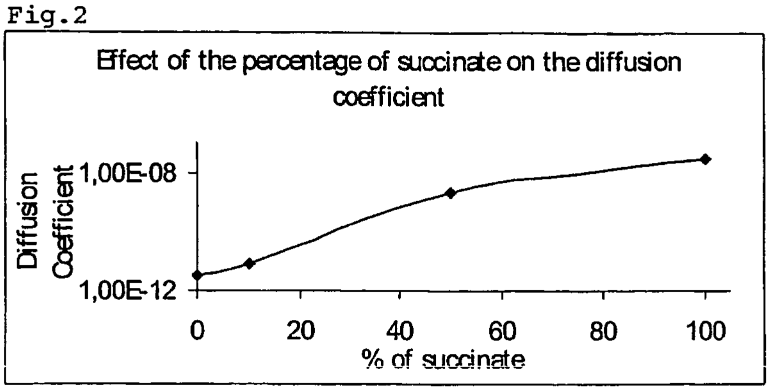


Graph 2: Effect of the percentage of succinate on the diffusion coefficient (for 0, 10, 50 and 100% of succinate).

Fig. 3

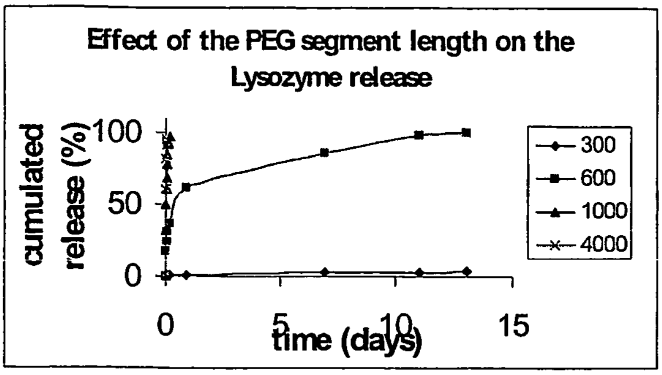


Graph 3: cumulated release (%) of lysozyme from 4 different PEG length copolymers: 300, 600, 1000 and 4000, when the T/S ratio is 50/50 mol % and when "b" is 60 and "c" is 40.

Fig. 4

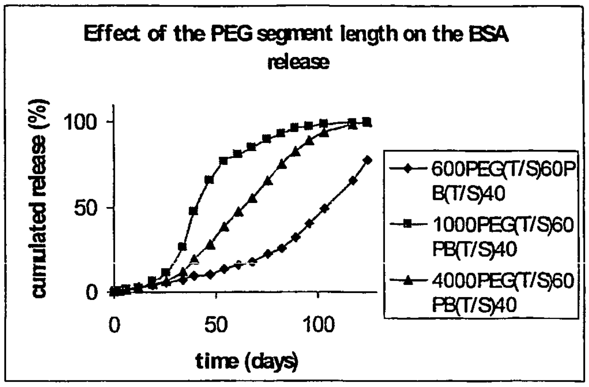


Graph 4: cumulated release (%) of BSA from 3 different PEG length copolymers: 600, 1000 and 4000, when the T/S ratio is 50-50 mol % and when "b" is 60 and "c" is 40.

Fig. 5

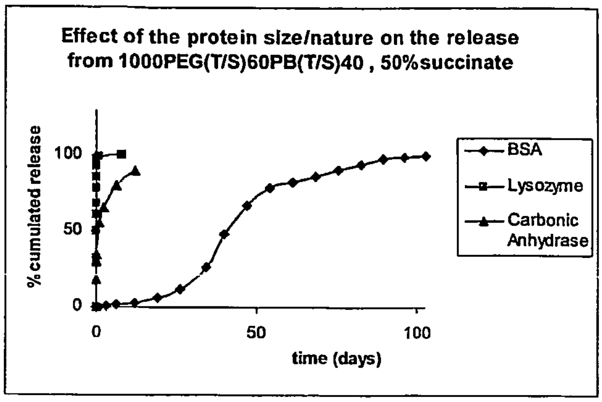


Graph 5: cumulated release (%) of BSA, Lysozyme and Carbonic Anhydrase from the 1000PEG(T/S)60PB(T/S)40, when the T/S ratio is 50/50 mol %.

Fig. 6

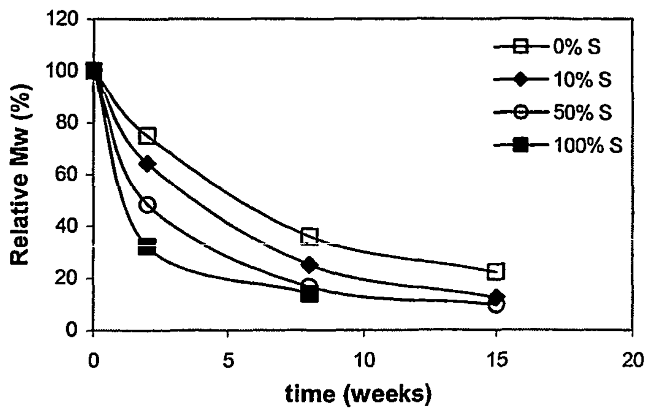


Graph 6: relative molecular weight for 1000PEG(T/S)65PB(T/S)35 (in vivo)

Fig. 7

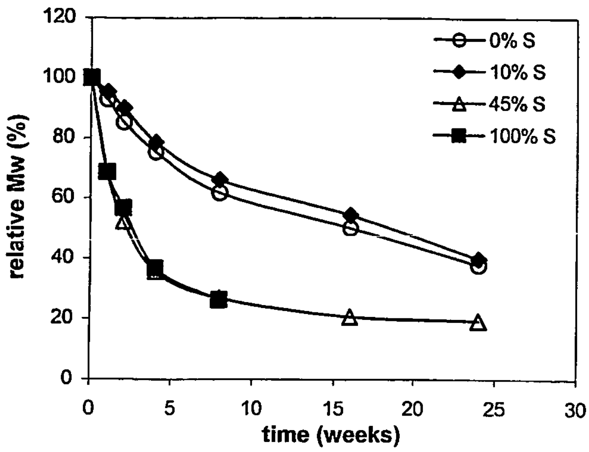


Graph 7: relative molecular weight for 1000PEG(T/S)65PB(T/S)35 (in vitro)